# EUROPEAN PATENT APPLICATION

(11) **EP 4 607 195 A1**
(43) Date of publication of application: **27.08.2025**
(21) Application number: 25156146.0
(22) Date of filing: 06.02.2025
(51) Int. Cl.: G01N 33/543, G01N 33/72

(54) **KIT FOR QUANTITATIVE ANALYSIS OF C-REACTIVE PROTEIN, METHOD FOR QUANTITATIVE ANALYSIS OF C-REACTIVE PROTEIN, AND APPARATUS FOR PERFORMING THE SAME**

(30) Priority: 26.02.2024 KR 20240027250
(71) Applicant: I-SENS, INC., Seoul 06646 (KR)
(72) Inventor: Kim, Ji Hoon, 06646 Seoul (KR); Choi, Dong Cheol, 06646 Seoul (KR); Park, Ji Yeon, 06646 Seoul (KR); Kim, Jin Young, 06646 Seoul (KR); Kim, Seung Wan, 06646 Seoul (KR); Kim, Hyeong Eun, 06646 Seoul (KR)
(74) Representative: Westphal, Mussgnug & Partner Patentanwälte mbB

(57) **Abstract**

The kit for quantitative analysis of C-Reactive Protein (CRP) according to an embodiment of the present application may comprises: a first composition including a hemolytic reagent for hemolyzing at least a portion of blood cells in a blood sample; and a second composition including an anti-CRP antibody for antigen-antibody reaction of CRP contained in the hemolyzed sample.

## Description

### CROSS-REFERENCE TO RELATED APPLICATION

This application claims priority to and the benefit of Korean Patent Application No. 10-2024-0027250, filed on February 26, 2024, the disclosure of which is incorporated herein by reference in its entirety.

### BACKGROUND

### 1. Field of the Invention

The present application relates to the quantitative analysis of biological samples and, more specifically, to a kit for quantitative analysis of C-Reactive Protein (CRP), a method for quantitative analysis of CRP, and/or a quantitative analysis apparatus for performing the same.

### 2. Discussion of Related Art

C-Reactive Protein (CRP) is a substance produced in the liver and secreted into the bloodstream within hours of an infection or inflammation. CRP levels in the blood have been widely utilized as a valuable marker for monitoring the treatment efficacy of infectious and autoimmune diseases. Meanwhile, with the publication of studies indicating that chronic inflammation serves as a mediator for the onset of type 2 diabetes, measuring CRP, an inflammation-related marker, has garnered attention for the development and research of technologies to predict the manifestation of type 2 diabetes. (Reference 1: The clinical significance and potential role of C-Reactive Protein in Chronic Inflammatory and Neurodegenerative Diseases, 2018; Reference 2: Inflammatory Markers and Risk of Type 2 Diabetes, 2013).

The prior art, as described in the following patent documents for quantitative analysis of CRP, quantified CRP in blood samples without hemolyzing red blood cells contained therein. However, the absence of hemolysis necessitated the separate determination of the hematocrit, which is the proportion of red blood cells in the blood sample. Additionally, to prevent hemolysis of red blood cells, further modifications to the substrate were required, making the CRP quantitative analysis process relatively complex. Furthermore, previous studies lacked sufficient investigation into stabilizers aimed at enhancing the storage stability of reagents included in CRP quantitative analysis kits.

Thus, there is a need for the development and research of CRP quantitative analysis technologies that improve usability and enhance the storage stability of reagents.

### [REFERENCE]

### [Patent Document]

[Patent Document 1] Korean Published Patent Application No. 10-2023-0038489 (March 20, 2023)

### SUMMARY OF THE INVENTION

The objectives of the present invention are to provide a kit for quantitative analysis of C-Reactive Protein (CRP), a method for quantitative analysis of CRP, and/or an apparatus for performing the same, which improve usability by utilizing a hemolytic reagent that does not affect the quantitative analysis of CRP.

The objectives of the present invention are to provide a kit for quantitative analysis of C-Reactive Protein (CRP), a method for quantitative analysis of CRP, and/or an apparatus for performing the same, which enhance the storage stability of reagents.

Objects of the present invention are not limited to the above-described objects and other objects that are not described may be clearly understood by those skilled in the art from this specification and the accompanying drawings.

The kit for quantitative analysis of C-Reactive Protein (CRP) according to an embodiment of the present application may comprises: a first composition including a hemolytic reagent for hemolyzing at least a portion of blood cells in a blood sample; and a second composition including an anti-CRP antibody for antigen-antibody reaction of CRP contained in the hemolyzed sample, wherein the kit for quantitative analysis of CRP comprises at least one reagent fixing part, the first composition and the second composition are independently fixed to the reagent fixing part, respectively, and the second composition further comprises a stabilizer for the stability of the anti-CRP antibody.

Solutions of the present invention are not limited to the above-described solutions and other solutions that are not described may be clearly understood by those skilled in the art from this specification and the accompanying drawings.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG 1 is a schematic diagram of a kit for quantitative analysis of C-Reactive Protein (CRP) according to an embodiment of the present application.
FIG 2 is a schematic diagram of a sample collector of the CRP quantitative analysis kit according to an embodiment of the present application.
FIG 3 is a schematic diagram of the main cartridge of the CRP quantitative analysis kit according to an embodiment of the present application.
FIG 4 is a schematic diagram of a solution cell of the CRP quantitative analysis kit according to an embodiment of the present application.
FIG 5 is a diagram illustrating the aspects where the reaction buffer stored in the solution cell flows into the mixing region of the main cartridge as the sample collector is inserted into the main cartridge, according to an embodiment of the present application.
FIG 6 is a diagram illustrating the aspect of antigen-antibody reactions used for quantitative analysis according to an embodiment of the present application.
FIGs. 7 and 8 are diagrams illustrating the detailed structure of the CRP quantitative analysis kit according to an embodiment of the present application.
FIG 9 is a diagram illustrating the analysis aspect for quantifying CRP in a blood sample according to an embodiment of the present application.
FIG 10 is a table showing the results of evaluating the hemolysis effect of different hemolytic reagents according to an embodiment of the present application.
FIG 11 is a graph showing the results of evaluating antigen-antibody reactivity for different hemolytic reagents according to an embodiment of the present application.
FIG 12 is a graph showing the results of evaluating antigen-antibody reactivity for different hemolytic reagents according to an embodiment of the present application.
FIG 13 is a graph and diagram showing the results of accelerated stability evaluation for stabilizers added to the Anti-CRP antibody according to an embodiment of the present application.
FIG 14 is a graph showing the results of accelerated stability evaluation for stabilizers added to the Anti-CRP antibody according to an embodiment of the present application.
FIG 15 is a graph showing the results of accelerated stability evaluation for stabilizers added to the Anti-CRP antibody according to an embodiment of the present application.
FIG 16 is a graph showing the results of accelerated stability evaluation for stabilizers added to the Anti-CRP antibody according to an embodiment of the present application.
FIG 17 is a diagram showing identification information included in the CRP quantitative analysis kit according to an embodiment of the present application.

### DETAILED DESCRIPTION OF EXEMPLARY EMBODIMENTS

The above-described objects, features and, advantages of the present invention will be clearly understood through the following detailed description taken in conjunction with the accompanying drawings. However, while the present invention can be modified in various ways and take on various alternative forms, specific embodiments thereof are shown in the drawings and described in detail below as examples.

Like reference numerals refer to like elements in principle throughout the specification. Further, elements with the same function within the scope of the same idea shown in the drawings of each embodiment will be described using the same reference numerals, and the descriptions thereof will not be repeated.

When it is determined that detailed descriptions of related well-known functions or configurations may unnecessarily obscure the gist of the present invention, detailed descriptions thereof will be omitted. Further, the ordinal numbers (for example, first, second, etc.) used in description of the specification are used only to distinguish one element from another element.

Further, the term "module," "unit," "part," or "portion" of an element used herein is assigned or incorporated for convenience of specification description, and the term itself does not have a distinct meaning or role.

As used herein, the singular forms "a" and "an" are intended to include the plural forms as well, unless the context clearly indicates otherwise.

It will be further understood that the terms "comprise," "comprising," "include," and/or "including" used herein specify the presence of stated features or elements, but do not preclude the presence or addition of one or more other features or elements.

Sizes of elements in the drawings may be exaggerated for convenience of explanation. In other words, since sizes and thicknesses of elements in the drawings are arbitrarily illustrated for convenience of explanation, the following embodiments are not limited thereto.

When a certain embodiment may be implemented differently, a specific process order may be performed differently from the described order. For example, two consecutively described processes may be performed substantially at the same time or performed in an order opposite to the described order.

In the following embodiments, when a first element is referred to as being "connected" to a second element, it includes not only a case where the two elements are "directly connected," but also a case where the two elements are "indirectly connected" with a third element interposed therebetween. For example, in this specification, when a first element is referred to as being "electrically connected" to a second element, it includes not only a case where the two elements are "directly electrically connected," but also a case where the two elements are "indirectly electrically connected" with a third element interposed therebetween.

The kit for quantitative analysis of C-Reactive Protein (CRP) according to an embodiment of the present application may comprises: a first composition including a hemolytic reagent for hemolyzing at least a portion of blood cells in a blood sample; and a second composition including an anti-CRP antibody for antigen-antibody reaction of CRP contained in the hemolyzed sample, wherein the CRP quantitative analysis kit for quantitative analysis of CRP comprises at least one reagent fixing part, the first composition and the second composition are independently fixed to the reagent fixing part, respectively, and the second composition further comprises a stabilizer for the stability of the anti-CRP antibody.

According to an embodiment of the present application, the hemolytic reagent may be selected from the group consisting of Sodium Deoxycholate (SDO) and Saponin.

According to an embodiment of the present application, the hemolytic reagent may be Sodium Deoxycholate (SDO).

According to an embodiment of the present application, the stabilizer may be selected from the group consisting of Trehalose and Sucrose.

According to an embodiment of the present application, the stabilizer may include both Trehalose and Sucrose.

According to an embodiment of the present application, the stabilizer may comprise Trehalose and Sucrose in substantially the same concentration ratio.

According to an embodiment of the present application, wherein the at least one reagent fixing part may comprise: a first reagent fixing part located in a first compartmental region of the CRP quantitative analysis kit, and a second reagent fixing part located in a second compartmental region partitioned from the first compartmental region, wherein the first composition may be fixed to the first reagent fixing part, and the second composition may be fixed to the second reagent fixing part.

According to an embodiment of the present application, wherein the CRP quantitative analysis kit may include a solution cell disposed in the first compartmental region, the solution cell may store a reaction buffer, and when a sample collector containing the blood sample is inserted into a receiving part of the CRP quantitative analysis kit, the reaction buffer stored in the solution cell and the blood sample contained in the sample collector may flow into a mixing region where the first reagent fixing part in the first compartmental region is disposed, so that at least a portion of blood cells in the blood sample may be hemolyzed in the mixing region.

According to an embodiment of the present application, wherein the reaction buffer may comprise Glycine, Sodium Chloride, Sodium Azide, Ethylenediaminetetraacetic acid disodium salt dihydrate, and Bovine Serum Albumin (BSA).

According to an embodiment of the present application, wherein the CRP quantitative analysis kit may further comprise a flow path connecting the first compartmental region and the second compartmental region, and the sample, in which at least a portion of the blood cells are hemolyzed, may flow from the mixing region in the first compartmental region to the second compartmental region through the flow path as the CRP quantitative analysis kit rotates by a predetermined angle due to an applied external force.

According to an embodiment of the present application, wherein the CRP quantitative analysis kit may further comprise a measurement unit for quantifying CRP contained in the blood sample, and the sample, in which at least a portion of the blood cells are hemolyzed, may pass through the measurement unit via the flow path while moving from the mixing region in the first compartmental region to the second reagent fixing part in the second compartmental region.

According to an embodiment of the present application, wherein the quantification of CRP may be performed based on a first turbidity measured through the measurement unit before the antigen-antibody reaction and a second turbidity measured through the measurement unit after the antigen-antibody reaction performed at the second reagent fixing part.

According to an embodiment of the present application, wherein the second composition may comprise the stabilizer for immobilizing the anti-CRP antibody in a solid state on the reagent fixing part.

According to an embodiment of the present application, wherein the anti-CRP antibody may be provided in a form coated on latex particles.

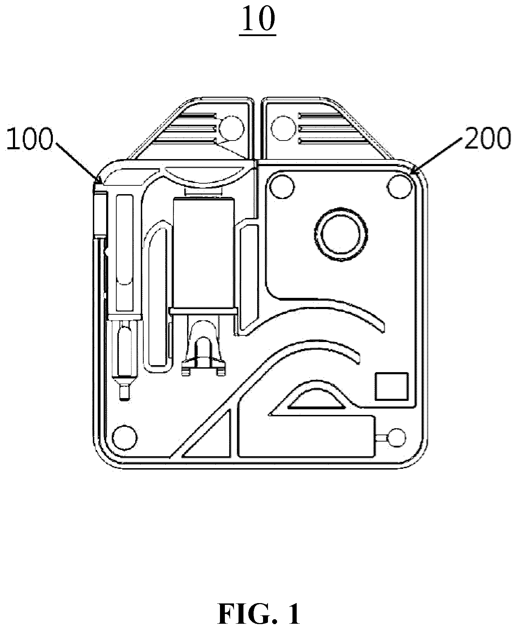

Hereinafter, with reference to Figures 1 to 17, the structure of the kit for quantitative analysis of C-Reactive Protein (CRP), the method for quantitative analysis of CRP, and/or the apparatus for quantitative analysis of CRP according to an embodiment of the present application will be described in greater detail.

FIG 1 is a schematic diagram of a kit for quantitative analysis of C-Reactive Protein (CRP) according to an embodiment of the present application.

The kit for quantitative analysis of C-Reactive Protein (hereinafter referred to as "CRP") 10 according to an embodiment of the present application may include a sample collector 100 for supplying a biological sample and/or a main cartridge 200 into which the sample collector 100 can be inserted and received.

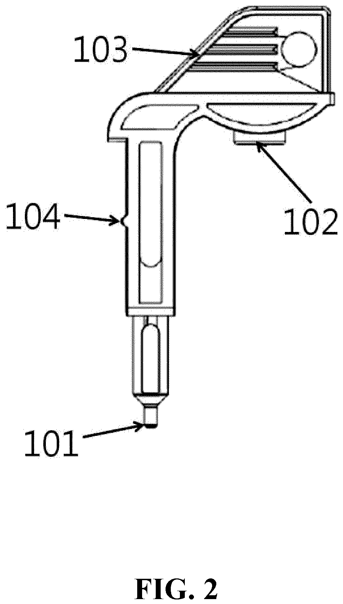

FIG 2 is a schematic diagram of a sample collector 100 of the CRP quantitative analysis kit according to an embodiment of the present application.

The sample collector 100 according to an embodiment of the present application may be configured to collect a predetermined amount of a biological sample (e.g., a blood sample) intended for analysis. Specifically, the sample collector 100 may include a capillary-type sample inlet 101 and may collect the biological sample (e.g., a blood sample) to be analyzed through the capillary provided in the sample inlet 101, injecting the collected biological sample into the main cartridge 200.

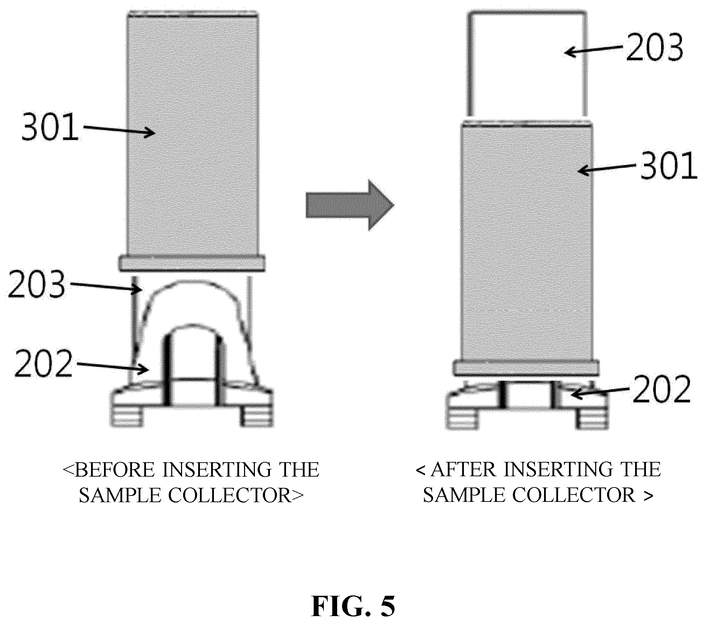

Furthermore, the sample collector 100 may include a protrusion 102 designed to contact a solution cell 301 inside the main cartridge 200 and push it inward when the sample collector 100 is inserted into the receiving part 201 of the main cartridge 200, described later. Specifically, the protrusion 102 may be positioned on the sample collector 100 to contact the solution cell 301 when the sample collector 100 is inserted into the receiving part 201 of the main cartridge 200. Consequently, as the sample collector 100 is inserted into the receiving part 201 of the main cartridge 200, the solution cell 301 may be moved inward toward the interior of the main cartridge 200, causing the cover tape of the solution cell 301 to be removed or ruptured. This will be described in more detail with reference to Fig. 5.

Additionally, the sample collector 100 may further include a handle 103 and/or a locking clip 104. Specifically, the handle 103 is a component designed to facilitate the transportation or use of the sample collector 100 and is not limited to the structure shown in Fig. 2. The locking clip 104 may be provided on one side of the sample collector 100 to secure the sample collector 100 to the main cartridge 200 when inserted into main cartridge 200. The locking clip 104 is designed to match the size and shape of a locking groove 211 provided in the receiving part 201 of the main cartridge 200. The locking clip 104 of the sample collector 100 and the locking groove 211 of the main cartridge 200 can engage with each other. As a result, the inserted sample collector 100 may be fixed to the main cartridge 200, preventing movement or detachment of the sample collector 100 even if the CRP quantitative analysis kit rotates during analysis.

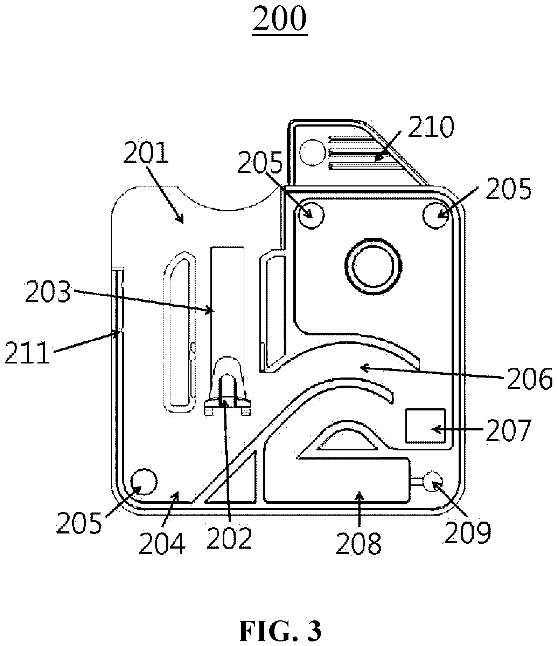

FIG 3 is a schematic diagram of the main cartridge 200 of the CRP quantitative analysis kit according to an embodiment of the present application.

The main cartridge 200 according to an embodiment of the present application may include a receiving part 201 into which the sample collector 100 can be inserted. Furthermore, the receiving part 201 may include a locking groove 211 configured to engage with the locking clip 104 of the sample collector 100, as described above, to secure the sample collector 100.

The main cartridge 200 may also include a moving frame 203 configured to fix and move the solution cell 301 provided inside the main cartridge. Specifically, the moving frame 203 may be configured to secure the solution cell 301 prior to the insertion of the sample collector 100. When the sample collector 100 is inserted, the solution cell 301, pushed by the protrusion 102 of the sample collector 100, may move along a moving path in the moving frame 203.

The main cartridge 200 may further include a cover tape rupturing part 202 configured to remove or rupture the cover tape 302 of the solution cell 301 as the solution cell 301 moves along the moving frame 203. This will be described in greater detail with reference to Fig. 5.

The main cartridge 200 may include a mixing part 204 (or mixing region) where the biological sample (e.g., a blood sample) discharged from the sample collector 100 through the receiving part 201 mixes with the reaction buffer discharged from the solution cell 301.

The main cartridge 200 may also include at least one reagent fixing part 205 (referred to as a sample fixing part) introduced with chemical reagents that can react with the reaction buffer and biological sample mixed in the mixing part 204 to induce enzymatic reaction and/or antigen-antibody reactions.

The main cartridge 200 may further include a flow path 206 through which the mixed reaction buffer and biological sample in the mixing part 204 can move. Specifically, the analysis sample may travel, through the flow path 206, between the mixing part 204, the reagent fixing part 205, and/or a measurement unit 207 that optically measures the analysis sample. The structure of the flow path 206 is not limited, provided it is designed to allow the analysis sample to move by gravity when the main cartridge 200 is tilted.

The main cartridge 200 according to an embodiment of the present application may include a measurement unit 207 for measuring the reaction results performed at the reagent fixing part 205. The CRP quantitative analysis apparatus according to an embodiment of the present application can quantify the analysis sample (e.g., CRP) through optical analysis, such as UV/VIS, via the measurement unit 207. For example, the measurement unit 207 of the main cartridge 200 can quantify the analysis sample (e.g., CRP) by measuring changes in turbidity (or absorbance) based on immunoturbidimetry.

The main cartridge 200 may include a waste solution treatment part 208 for collecting waste solutions which analysis was completed via the measurement unit 207. The waste solution treatment part 208 allows for the collection and separate disposal of waste solutions, which are a type of medical waste. Collection of waste solutions via the waste solution treatment part 208 can be achieved by absorbing the waste solution into highly absorbent cotton, absorbent filters, or polymer-based absorbent materials, placed in the waste solution treatment part 208.

The main cartridge 200 may further include an air vent 209 to facilitate the smooth movement and absorption of the waste solution into the absorbent materials. Through the air vent 209, the transfer of waste solutions to the waste solution treatment part 208 and their subsequent collection can be more efficiently performed.

The main cartridge 200 may also include a handle 210 to facilitate its transportation and use. The structure of the handle 210 is not limited to the form shown in Fig. 3.

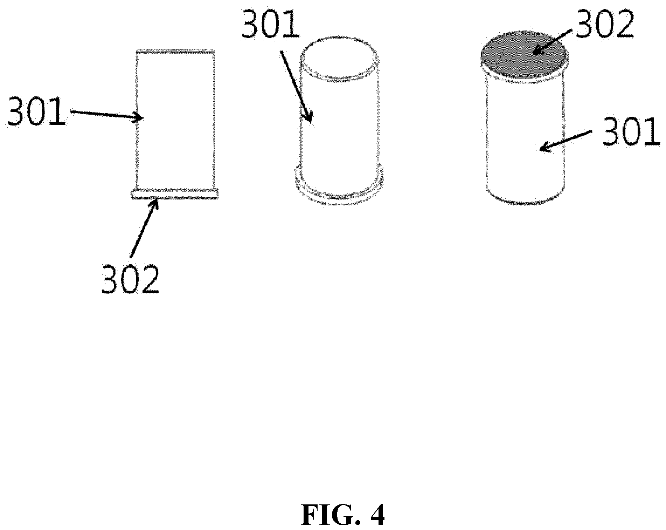

Hereinafter, with reference to Figures 4 and 5, a more detailed description will be provided of the aspects in which the reaction buffer of the solution cell 301 flows into the mixing part 204 of the main cartridge 200 when the sample collector 100 is inserted into the receiving part 201 of the main cartridge 200. FIG 4 is a schematic diagram of a solution cell 301 of the CRP quantitative analysis kit according to an embodiment of the present application. FIG 5 is a diagram illustrating the aspects where the reaction buffer stored in the solution cell 301 flows into the mixing region of the main cartridge 200 as the sample collector 100 is inserted into the main cartridge 200, according to an embodiment of the present application.

The main cartridge 200 according to an embodiment of the present application may include a solution cell 301 that stores a reaction buffer for reacting with the biological sample from the sample collector 100. The solution cell 301 includes an opening at one end, sealed with a cover tape 302, to prevent the reaction buffer stored inside from leaking. The opening sealed with the cover tape 302 of solution cell 301 is positioned to face the cover tape rupturing part 202 of the main cartridge 200.

Before the sample collector 100 is inserted into the receiving part 201 of the main cartridge 200, the opening of the solution cell 301 sealed with the cover tape 302 may be positioned apart from the cover tape rupturing part 202. Before the sample collector 100 is inserted into the receiving part 201 of the main cartridge 200, the opening of the solution cell 301 sealed with the cover tape 302 may be positioned apart from the cover tape rupturing part 202. When the sample collector 100 is inserted into the receiving part 201 of the main cartridge 200, the protrusion 102 of the sample collector 100 applies pressure to the opposite end of the solution cell 301, away from the opening sealed with the cover tape 302. This applied pressure causes the solution cell 301 to move along the moving path of the moving frame 203.

As a result, when the cover tape 302 of the solution cell 301 comes into contact with the cover tape rupturing part 202, the cover tape 302 of the solution cell 301 is removed or ruptured. The reaction buffer stored in the solution cell 301 is then transferred through a flow path or hollow structure formed in the cover tape rupturing part 202 into the mixing part 204 of the main cartridge 200. In the mixing part 204, the reaction buffer mixes with the biological sample (e.g., a blood sample) supplied from the sample collector 100.

Specifically, the reaction buffer flowing into the mixing part 204 may be designed to contact the sample inlet 101 of the sample collector 100. This contact allows the biological sample contained in the capillary-type sample inlet 101 to move through the sample inlet 101 into the mixing part 204 of the main cartridge 200. Consequently, in the mixing part 204 of the main cartridge 200, the reaction buffer stored in the solution cell 301 and the biological sample collected by the sample collector 100 can be mixed.

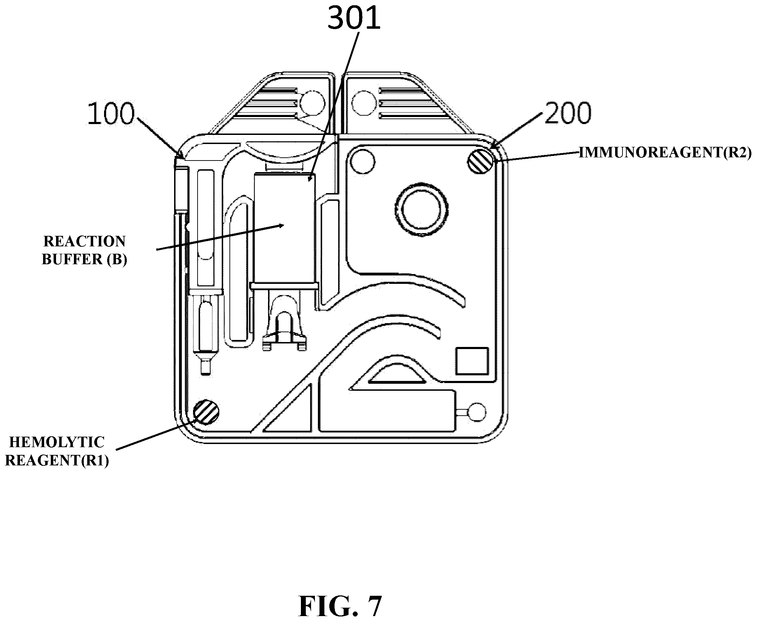

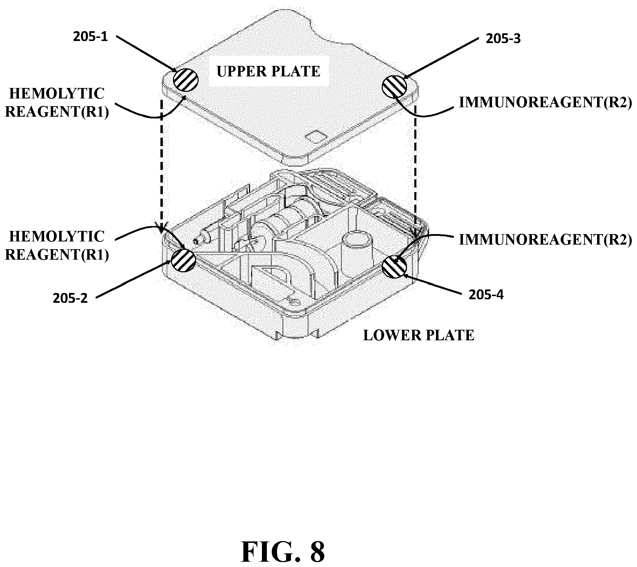

Furthermore, at least a portion of the blood cells contained in the biological sample (e.g., blood sample) may be hemolyzed through a composition fixed on the reagent fixing part 205 provided in the mixing part 204. This process will be described in greater detail with reference to Figures 7 and 8.

In Figures 4 and 5, the shape of the solution cell 301 is illustrated as a specific example. However, this is merely an example, and the shape of the solution cell 301 is not limited as long as it provides a structure suitable for storing the reaction buffer. Similarly, the material of the cover tape 302 is not particularly limited as long as it prevents the reaction buffer from leaking while allowing for easy removal or rupture.

Additionally, Figure 5 illustrates the cover tape rupturing part 202 in a specific shape for explanation purposes. However, this is also merely an example, and the shape of the cover tape rupturing part 202 is not limited, provided it facilitates the removal or rupture of the cover tape. Suitable structures include, but are not limited to, needle-shaped or edge-shaped configurations.

The CRP quantitative analysis kit 10 according to an embodiment of the present application may be used to quantitatively analyze CRP present in blood (e.g., plasma, serum, and/or whole blood). According to one embodiment, the CRP quantitative analysis kit 10 may be configured to quantitatively analyze CRP using an immunoassay. Specifically, the CRP quantitative analysis kit 10 may be configured to quantitatively analyze CRP using a turbidimetric immunoassay. Hereinafter, with reference to Figures 7 to 9, a more detailed description will be provided of the aspects of quantitatively analyzing CRP using the CRP quantitative analysis kit 10 according to an embodiment of the present application.

The CRP quantitative analysis according to an embodiment of the present application may utilize a turbidimetric immunoassay.

The turbidimetric immunoassay employs an antigen-antibody reaction. Specifically, an antibody corresponding to the target analyte in the analysis sample is added to the analysis sample, causing an antigen-antibody complex to form via the antigen-antibody reaction. The principle of the turbidimetric immunoassay is to measure the turbidity of the antigen-antibody complex, as the turbidity is proportional to the amount of antigen in the test substance, and to use this measurement to determine the concentration of the target analyte.

According to an embodiment of the present application, the CRP quantitative analysis kit 10 includes a composition containing an Anti-CRP antibody corresponding to the target analyte, CRP (antigen). In this case, a complex is formed through agglutination between the CRP antigen and the Anti-CRP antibody, and the formation of this complex leads to changes in turbidity. Therefore, the concentration of the target analyte, CRP, in the blood sample can be quantified based on the change in turbidity.

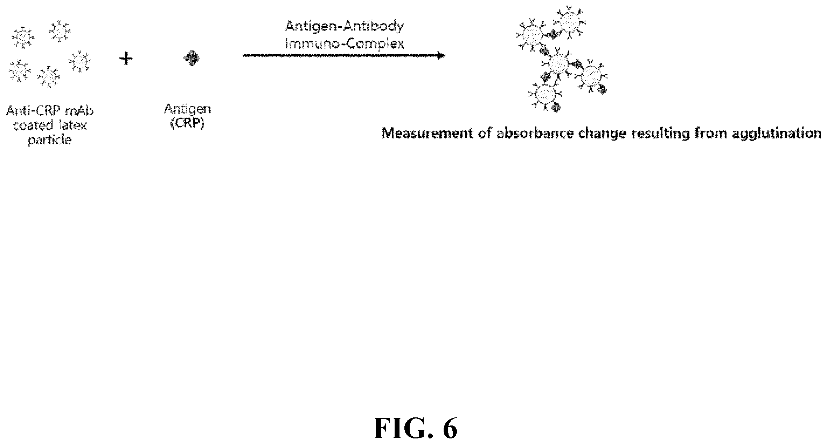

According to an embodiment of the present application, the CRP is quantitatively analyzed by implementing the antigen-antibody reaction of FIG 6. FIG 6 is a diagram illustrating the aspect of antigen-antibody reactions used for quantitative analysis according to an embodiment of the present application.

FIGs. 7 and 8 are diagrams illustrating the detailed structure of the CRP quantitative analysis kit 10 according to an embodiment of the present application.

The solution cell 301 of the CRP quantitative analysis kit 10 according to an embodiment of the present application may store a reaction buffer (B). As described above, the reaction buffer (B) stored in the solution cell 301 moves into the mixing part 204 of the main cartridge 200 as the sample collector 100 is inserted. At this time, the reaction buffer (B) mixes in the mixing part 204 with the blood sample collected through the sample collector 100 and with a composition (hereinafter referred to as the first composition) containing a hemolytic reagent (R1) fixed on at least one reagent fixing part 205. During the mixing process, the hemolytic reagent (R1) may hemolyze at least a portion of the blood cells contained in the blood sample. The reaction buffer (B) according to one embodiment may include glycine, sodium chloride, sodium azide, ethylenediaminetetraacetic acid disodium salt dihydrate, and bovine serum albumin (BSA).

The hemolytic reagent (R1) according to one embodiment may be selected from the group consisting of Sodium Deoxycholate (SDO), ASB-14, Saponin, and Triton X-100 (Tx-100). In another embodiment, the hemolytic reagent (R1) may be selected from the group consisting of Sodium Deoxycholate (SDO) and Saponin. In a preferred embodiment, the hemolytic reagent (R1) may be Sodium Deoxycholate (SDO). According to the CRP quantitative analysis kit, method for quantitative analysis of CRP, and/or apparatus for performing the same in a preferred embodiment of the present application, the use of Sodium Deoxycholate (SDO) as the hemolytic reagent (R1) enables the quantitative analysis of CRP with high precision over a wide range of CRP concentrations without affecting the immune reaction. However, this is merely an example, and the hemolytic reagent (R1) is not limited thereto. Any suitable composition or surfactant (detergent) capable of achieving the purpose of hemolyzing blood cells without affecting the immune reaction (antigen-antibody reaction) may be used as the hemolytic reagent (R1).

The sample, in which at least a portion of the blood cells present in the blood sample has been hemolyzed by the hemolytic reagent (R1), may move through the flow path 206 to the measurement unit 207 and/or at least one reagent fixing part 205. The at least one reagent fixing part 205 may have a composition (hereinafter referred to as the second composition) containing an immunoreagent (R2) for the antigen-antibody reaction (or immune reaction) of CRP present in the hemolyzed sample. According to one embodiment, the immunoreagent (R2) may include an Anti-CRP antibody. In a preferred embodiment, the Anti-CRP antibody of the immunoreagent (R2) may be provided in a form coated on latex particles.

According to an embodiment of the present application, the first composition containing the hemolytic reagent (R1) and the second composition containing the immunoreagent (R2) may each be independently fixed to at least one reagent fixing part 205. In one embodiment, the first composition containing the hemolytic reagent (R1) may be fixed to a first reagent fixing part 205 located in a first compartmental region (e.g., the region present in the mixing part 204). Meanwhile, the second composition containing the immunoreagent (R2) may be fixed to a second reagent fixing part 205 located in a second compartmental region partitioned from the first compartmental region.

Referring to FIG 8, the CRP quantitative analysis kit 10 may include a body comprising an upper plate and a lower plate configured to face each other. In this configuration, the first composition containing the hemolytic reagent (R1) may be dispensed and dried on a first reagent fixing part 205-1 provided in the first compartmental region of the upper plate of the body and/or a first reagent fixing part 205-2 provided in the first compartmental region of the lower plate of the body. Furthermore, the first reagent fixing part 205-1 provided on the upper plate of the body and the first reagent fixing part 205-2 provided on the lower plate of the body may be configured to face each other.

Meanwhile, the second composition containing the immunoreagent (R2) may be dispensed and dried on a second reagent fixing part 205-3 located in the second compartmental region of the upper plate of the body and/or a second reagent fixing part 205-4 located in the second compartmental region of the lower plate of the body. Furthermore, the second reagent fixing part 205-3 provided on the upper plate of the body and the second reagent fixing part 205-4 provided on the lower plate of the body may be configured to face each other.

As described above, the first compartmental region, where the first reagent fixing part 205-1 and/or 205-2 securing the hemolytic reagent (R1) is located, may be partitioned from the second compartmental region, where the second reagent fixing part 205-3 and/or 205-4 securing the immunoreagent (R2) is located. The first compartmental region and the second compartmental region may be connected via a flow path 206.

According to an embodiment of the present application, the first composition including the hemolytic reagent (R1) and/or the second composition including the immunoreagent (R2) may be fixed in a solid state on the reagent fixing part 205. Specifically, the first composition including the hemolytic reagent (R1) and/or the second composition including the immunoreagent (R2) may be dispensed onto and fixed on at least one reagent fixing part 205 in a dried state.

According to an embodiment of the present application, the second composition including the immunoreagent (R2) (and/or the first composition including the hemolytic reagent (R1)) may further include a stabilizer to enhance the stability of the immunoreagent (R2) (and/or the hemolytic reagent (R1)) and to fix the reagent in a solid state on the reagent fixing part 205.

According to an embodiment of the present application, the stabilizer may be selected from the group consisting of trehalose and sucrose. In a preferred embodiment, the stabilizer may include both trehalose and sucrose. In a more preferred embodiment, the stabilizer may include trehalose and sucrose in substantially the same concentration ratio.

According to the CRP quantitative analysis kit, method for quantitative analysis of CRP, and/or apparatus for performing the same in an embodiment of the present application, the use of a stabilizer containing sucrose and trehalose in an optimal concentration ratio provides the following benefits:
1) The Anti-CRP antibody, dried in a solid state, can enoughly rehydrate and participate in the immune reaction.
2) Quantitative analysis of CRP can be performed with high precision and accuracy over a wide range of CRP concentrations.
3) Storage stability of the reagents can be enhanced.

However, this is merely an example, and any suitable material capable of enhancing the stability of the immunoreagent while improving the precision and accuracy of CRP quantitative analysis may be used as the stabilizer.

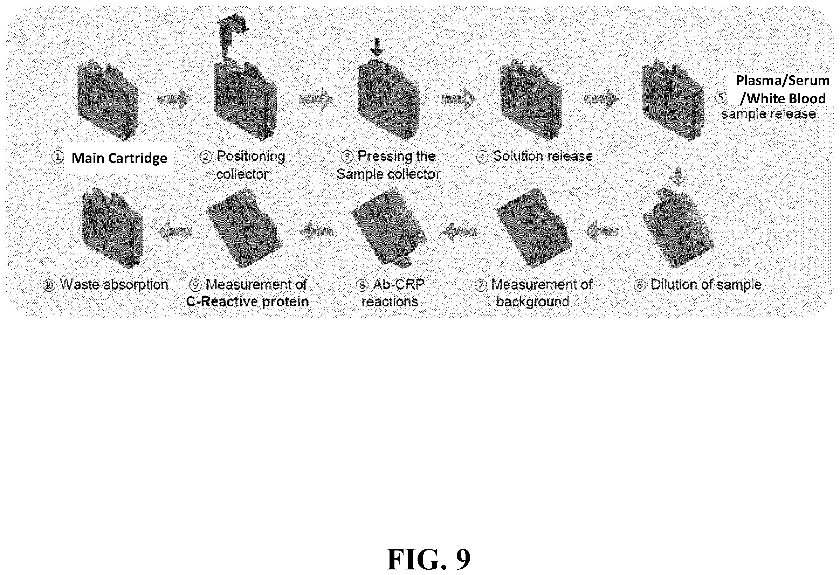

FIG 9 is a diagram illustrating the analysis aspect for quantifying CRP in a blood sample according to an embodiment of the present application.

Referring to FIG 9, the CRP quantitative analysis method according to an embodiment of the present application may be implemented as follows:
1) Prepare the main cartridge 200.
2) Position the sample collector 100 on the receiving part 201 of the main cartridge 200.
3) Apply pressure to the sample collector 100 in the direction toward the receiving part 201 of the main cartridge 200.
4) As the protrusion 102 of the sample collector 100 applies pressure to the solution cell 301, the solution cell 301 moves, as described above, through the moving frame 203 in the direction of the cover tape rupturing part 202. The cover tape rupturing part 202 comes into contact with the cover tape 302 of the solution cell 301, causing the solution (reaction buffer) stored inside the solution cell 301 to flow into the mixing part 204 of the main cartridge 200.
5) As the reaction buffer flows into the mixing part 204, it comes into contact with the blood sample from the sample collector 100, causing the blood sample (e.g., plasma, serum, and/or whole blood) to also flow into the mixing part 204 of the main cartridge 200.
6) In the mixing part 204, the hemolytic reagent (R1) fixed on the first reagent fixing part 205-1 and/or 205-2 located in the mixing part 204, the reaction buffer (B), and the blood sample are mixed. Furthermore, the hemolytic reagent (R1) hemolyzes at least a portion of the blood cells in the blood sample.

Meanwhile, according to an embodiment of the present application, the CRP quantitative analysis kit 10 rotates by a predetermined angle due to an external force (e.g., rotational force applied by a quantitative analysis device, as described later). Consequently, the blood sample inside the main cartridge 200 moves through the flow path 206 of the main cartridge 200 under the influence of gravity.

7) Due to the external force applied to the CRP quantitative analysis kit 10, the sample, in which at least a portion of the blood cells has been hemolyzed, moves to the measurement unit 207. While the sample is positioned on the measurement unit 207, a background measurement value (first turbidity) related to the turbidity (or absorbance) before the antigen-antibody reaction is performed can be measured.

As described above, the CRP quantitative analysis kit 10 may include a measurement unit 207 for quantifying CRP contained in the blood sample. According to one embodiment, the CRP quantitative analysis kit 10 may be configured such that the hemolyzed sample passes through the measurement unit 207 while moving through the flow path 206 from the mixing part 204, where the first reagent fixing part securing the hemolytic reagent (R1) is located, to the second reagent fixing part securing the immunoreagent (R2). In this embodiment, the turbidity before and after the antigen-antibody reaction can be easily measured, allowing the CRP concentration, which correlates with the change in turbidity, to be quantified more easily.

8) Due to the external force applied to the CRP quantitative analysis kit 10, the hemolyzed sample moves from the measurement unit 207 to the second reagent fixing part 205-3 and/or 205-4, where the immunoreagent (R2) is fixed. On the second reagent fixing part 205-3 and/or 205-4, an antigen-antibody reaction (Ab-CRP Reaction in Figure 8) occurs between the CRP (antigen) contained in the sample and the Anti-CRP antibody included in the immunoreagent (R2). Through this antigen-antibody reaction, the CRP antigen forms an immune complex (Immuno-Complex) with the Anti-CRP antibody, causing a change in the turbidity of the sample.

9) Due to the external force applied to the CRP quantitative analysis kit 10, the sample in which the antigen-antibody reaction has occurred moves from the second reagent fixing part 205-3 and/or 205-4 to the measurement unit 207. While the sample is positioned on the measurement unit 207, the turbidity (or absorbance) of the sample after the antigen-antibody reaction, referred to as the second turbidity, can be measured. Furthermore, the CRP can be quantified based on the first turbidity measured by the measurement unit 207 before the antigen-antibody reaction and the second turbidity measured by the measurement unit 207 after the antigen-antibody reaction.

10) The waste from the completed quantitative analysis moves from the measurement unit 207 to the waste solution treatment part 208 due to the external force applied to the CRP quantitative analysis kit 10, where it is collected.

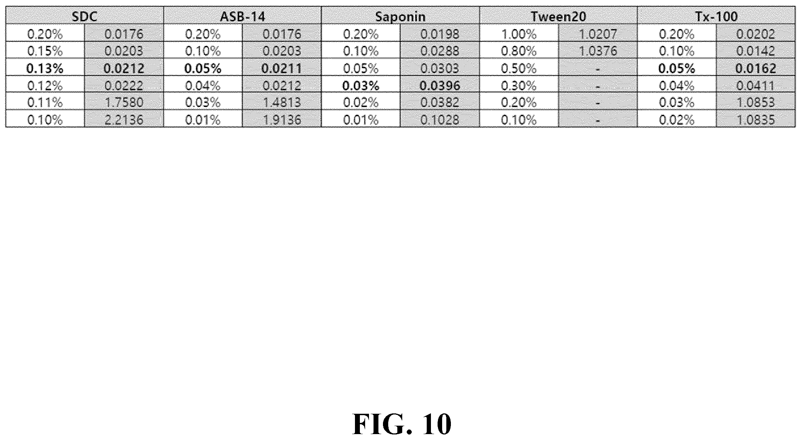

Hereinafter, with reference to FIGS. 10 to 16, the present invention will be described in detail through experimental examples. However, the following experimental examples are merely illustrative and should not be construed as limiting.

### <Experimental Example 1: Evaluation of Hemolytic Effect of Hemolytic Reagents>

### 1. Experimental Method

To select hemolytic reagents with a hemolytic effect capable of lysing the blood cells in a blood sample, five candidate hemolytic reagents were selected. Each candidate hemolytic reagent was applied to fresh venous whole blood samples (HCT 48%). The background measurement values (i.e., the first turbidity or first absorbance) of samples treated with each candidate hemolytic reagent were measured. Specifically, the first absorbance of the samples was measured at a wavelength of 750 nm using the A1Care Analyzer by i-SENS, Inc.

The five candidate hemolytic reagents are listed below Table 1. Table 1 shows five candidate hemolytic reagents and the evaluation concentrations of the candidate hemolytic reagents. A candidate hemolytic reagent was evaluated to have a hemolytic effect on the blood sample if the first turbidity of the sample was below 0.05.

**Table 1: Candidate hemolytic reagents and the evaluation concentrations of the candidate hemolytic reagents.**

| **Hemolytic Reagents** | **Sodium deoxycholate(SDC)** | **ASB-14** | **Saponin** | **Tween20** | **Triton X-100 (Tx-100)** |
|---|---|---|---|---|---|
| **Evaluation Concentration (w/v%)** | 0.2% ~ 0.1 % | 0.2% ~ 0.01% | 0.2% ~ 0.01% | 1.0% ~ 0.1% | 0.2% ~ 0.02% |

### 2. Experimental Results

FIG 10 is a table showing the results of evaluating the hemolysis effect of different hemolytic reagents according to an embodiment of the present application.

For the SDC hemolytic reagent, in the concentration range of 0.12-0.20 (w/v%), the first absorbance was measured to be less than 0.05, while in the concentration range of 0.10-0.11 (w/v%), the first absorbance was measured to be greater than 0.05.

For the ASB-14 hemolytic reagent, in the concentration range of 0.04-0.20 (w/v%), the first absorbance was measured to be less than 0.05, while in the concentration range of 0.01-0.03 (w/v%), the first absorbance was measured to be greater than 0.05.

For the Saponin hemolytic reagent, in the concentration range of 0.02-0.20 (w/v%), the first absorbance was measured to be less than 0.05, while at a concentration of 0.01 (w/v%), the first absorbance was measured to be greater than 0.05.

For the Tween20 hemolytic reagent, in the concentration range below 1.00 (w/v%), the first absorbance was measured to be greater than 0.05.

For the Tx-100 hemolytic reagent, in the concentration range of 0.04-0.20 (w/v%), the first absorbance was measured to be less than 0.05, while in the concentration range of 0.02-0.03 (w/v%), the first absorbance was measured to be greater than 0.05.

Through this experimental example, it was confirmed that the Tween20 hemolytic reagent exhibited relatively low hemolytic effects on the blood cells in the blood sample, even at high concentrations (1.00 w/v%). In contrast, the SDC, ASB-14, Saponin, and Tx-100 hemolytic reagents exhibited hemolytic effects on the blood cells in most concentration ranges. Therefore, the hemolytic reagent (R1) according to one embodiment of the present application may be selected from the group consisting of Sodium Deoxycholate (SDO), ASB-14, Saponin, and Triton X-100 (Tx-100).

### <Experimental Example 2: Evaluation of Antigen-Antibody Reactivity by Hemolytic Reagents>

### 1. Experimental Method

To select hemolytic reagents that do not interfere with the immune reaction between CRP and Anti-CRP antibodies, four candidate hemolytic reagents (SDC, ASB-14, Saponin, Tx-100) with confirmed hemolytic effects were prepared, as identified in Experimental Example 1. The concentrations of each candidate hemolytic reagent were determined as follows Table 2, taking into account the sample influence, including hematocrit. Table 2 shows the experimental conditions according to the type and concentration of hemolytic reagent.

**Table 2: Experimental Conditions according to the type and concentration of hemolytic reagent.**

| **Conditions** | **Control** | **1** | **2** | **3** | **4** |
|---|---|---|---|---|---|
| **Hemolytic Reagents** | - | 0.13% | 0.05% | 0.03% | 0.05% |
| | | **Sodium deoxycholate(SDC)** | **ASB-14** | **Saponin** | **Triton X-100 (Tx-100)** |

Furthermore, each candidate hemolytic reagent was applied to 7-Level CRP Serum Samples, followed by an immune reaction between the hemolyzed samples and latex particles coated with Anti-CRP antibodies. Specifically, latex particles coated with Anti-CRP antibodies supplied by RANDOX were concentrated and stabilized with the addition of a stabilizer. The immune reaction was configured to occur between the hemolyzed samples and the latex particles coated with Anti-CRP antibodies.

After the immune reaction, the absorbance of each sample after the immune reaction (referred to as the second absorbance or second turbidity) was measured. Specifically, the second absorbance of the samples was measured at a wavelength of 750 nm using the A1Care Analyzer by i-SENS, Inc.

As for the control group, the experiment was designed to measure the second absorbance after the immune reaction between the 7-Level CRP Serum Samples without the addition of any hemolytic reagent and the latex particles coated with Anti-CRP antibodies. Additionally, the concentrations of the 7-Level CRP Serum Samples were varied from 0 to 195.8 mg/L, and the second absorbance was measured for each sample.

### 2. Experimental Results

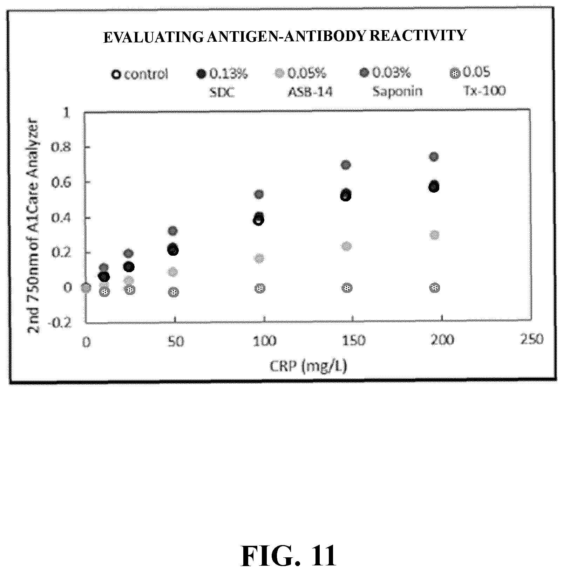

FIG 11 is a graph showing the results of evaluating antigen-antibody reactivity for different hemolytic reagents according to an embodiment of the present application.

For the SDC hemolytic reagent, it was confirmed that the absorbance was similar to that of the control group, where no hemolytic reagent was added (i.e., the immune reaction was not affected by the hemolytic reagent). In other words, for the SDC hemolytic reagent, it was confirmed that the hemolytic reaction did not affect the antigen-antibody reaction between CRP and Anti-CRP antibodies.

For the ASB-14 hemolytic reagent, it was confirmed that the absorbance corresponding to the CRP concentration decreased by approximately 50% or more compared to the control group, where no hemolytic reagent was added (i.e., the immune reaction was not affected by the hemolytic reagent).

For the Saponin hemolytic reagent, it was confirmed that the absorbance corresponding to the CRP concentration was relatively high compared to the control group, where no hemolytic reagent was added (i.e., the immune reaction was not affected by the hemolytic reagent). In other words, for the Saponin hemolytic reagent, it was confirmed that the hemolytic reaction did not affect the antigen-antibody reaction between CRP and Anti-CRP antibodies.

For the Tx-100 hemolytic reagent, it was observed that the measured absorbance did not show any correlation with the CRP concentration.

Through this experimental example, it was confirmed that the hemolytic reagent (R1) according to a preferred embodiment of the present application may be selected from the group consisting of Sodium Deoxycholate (SDO) and Saponin.

### <Experimental Example 3: Evaluation of Antigen-Antibody Reactivity by Hemolytic Reagents (2)>

### 1. Experimental Method

To select a hemolytic reagent that exhibits excellent precision while not affecting the immune reaction between CRP and Anti-CRP antibodies, two candidate hemolytic reagents (SDC and Saponin) that were confirmed in Experimental Example 2 to not interfere with the immune reaction through hemolytic reaction were prepared as follows Table 3. Table 3 shows the experimental conditions according to the type and concentration of the selected hemolytic reagent.

**Table 3: Experimental conditions according to the type and concentration of the selected hemolytic reagent.**

| **Conditions** | **Control** | **1** | **2** |
|---|---|---|---|
| **Hemolytic Reagents** | - | 0.13% | 0.03% |
| | | **Sodium deoxycholate(SDC)** | **Saponin** |

Furthermore, each candidate hemolytic reagent was applied to 1) CRP Control Solution (Low & High) and 2) 3-Level CRP Venous Whole Blood Samples (Low, Mild, High) respectively. Following the application, an immune reaction was conducted between the hemolyzed samples and latex particles coated with Anti-CRP antibodies. After the immune reaction, the absorbance of each sample after the immune reaction (i.e., the second absorbance) was measured. Specifically, the second absorbance of the samples was measured at a wavelength of 750 nm using the A1Care Analyzer by i-SENS, Inc.

Regarding the control group, the experiment was designed to measure the second absorbance after the immune reaction between the CRP Control Solution (Low & High) without any hemolytic reagent added and the latex particles coated with Anti-CRP antibodies.

Additionally, for each sample, at each concentration of the each sample, and for each hemolytic reagent, the second absorbance was measured 20 times, and the average (AVG), standard deviation (SD), and precision (CV) of the measured second absorbance values were calculated.

### 2. Experimental Results

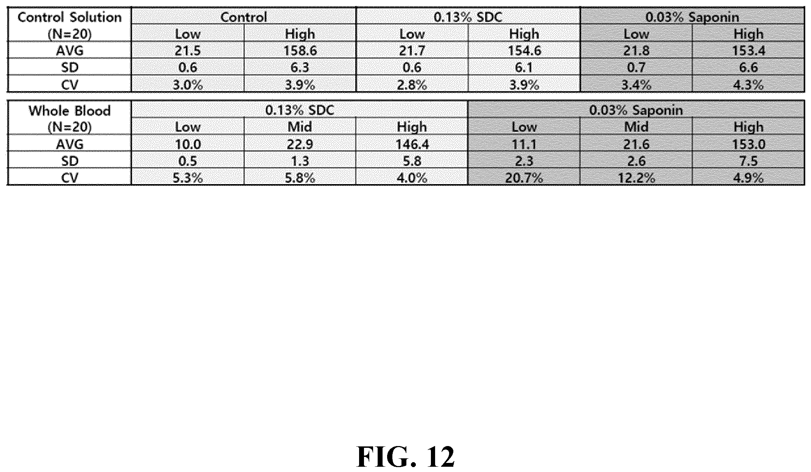

FIG 12 is a graph showing the results of evaluating antigen-antibody reactivity for different hemolytic reagents according to an embodiment of the present application.

For the CRP Control Solution (Low) sample, the precision (CV) of the SDC hemolytic reagent was measured at 2.8%, the precision (CV) of the Saponin hemolytic reagent was measured at 3.4%, and the precision (CV) of the control group was measured at 3.0%. It was confirmed that both the SDC and Saponin hemolytic reagents exhibited a similar level of precision compared to the control group.

For the CRP Control Solution (High) sample, the precision (CV) of the SDC hemolytic reagent was measured at 3.9%, the precision (CV) of the Saponin hemolytic reagent was measured at 4.3%, and the precision (CV) of the control group was measured at 3.9%. It was confirmed that both the SDC and Saponin hemolytic reagents exhibited a similar level of precision compared to the control group.

For the CRP Venous Whole Blood (Low) sample, the precision (CV) of the SDC hemolytic reagent was measured at 5.3%, while the precision (CV) of the Saponin hemolytic reagent was measured at 20.7%.

For the CRP Venous Whole Blood (Mid) sample, the precision (CV) of the SDC hemolytic reagent was measured at 5.8%, while the precision (CV) of the Saponin hemolytic reagent was measured at 12.2%.

For the CRP Venous Whole Blood (High) sample, the precision (CV) of the SDC hemolytic reagent was measured at 4.0%, while the precision (CV) of the Saponin hemolytic reagent was measured at 4.9%.

Thus, for the 3-Level CRP Whole Blood samples, it was confirmed that the precision of the SDC hemolytic reagent was superior to that of the Saponin hemolytic reagent across all concentration ranges of the 3-Level CRP Whole Blood samples.

According to this experimental example, the hemolytic reagent (R1) in a preferred embodiment of the present application may be Sodium Deoxycholate (SDO).

Furthermore, the CRP quantitative analysis kit 10 according to one embodiment of the present application may exhibit a precision (CV) of less than 10%. For example, the CRP quantitative analysis kit 10 may exhibit a precision (CV) of less than 8%. In another example, the CRP quantitative analysis kit 10 may exhibit a precision (CV) of less than 6%.

### <Experimental Example 4: Evaluation of Accelerated Stability by Stabilizers (1)>

### 1. Experimental Method

To select a stabilizer that ensures the storage stability of the immunoreagent (R2) containing Anti-CRP antibodies, five candidate stabilizers were chosen. Each candidate stabilizer was added to latex particles coated with Anti-CRP antibodies and dried. The reagent solution and 1-Level CRP Serum Sample (CRP 60 mg/L) were added to the dried composition to facilitate the immune reaction between CRP and Anti-CRP antibodies. After the immune reaction, the absorbance was measured to obtain the baseline measurement value for storage on day 0. The reagent solution was configured to include glycine (Glycine), sodium chloride (Sodium Chloride), sodium azide (Sodium Azide), ethylenediaminetetraacetic acid disodium salt dihydrate (Ethylenediaminetetraacetic acid disodium salt dihydrate), and bovine serum albumin (Bovine Serum Albumin, BSA).

Furthermore, the dried compositions, containing each candidate stabilizer added to latex particles coated with Anti-CRP antibodies, were stored in an oven at 50°C. The reagent solution was stored separately under refrigeration. At 1-day intervals over a period of 7 days, the reagent solution and 1-Level CRP Serum Sample were added to the dried compositions containing each candidate stabilizer. The immune reaction between CRP and Anti-CRP antibodies was carried out, and the absorbance after the immune reaction was measured to obtain the measurement values for storage days 1, 2, 3, 4, 5, 6, and 7.

As for the control group, the experiment was configured to measure the absorbance after the immune reaction between the 1-Level CRP Serum Sample (CRP 60 mg/L) without the addition of any stabilizer and latex particles coated with Anti-CRP antibodies.

Furthermore, deviations (Bias) were calculated based on the baseline measurement value and the measurement values obtained over the storage period.

The five candidate stabilizers are as follows Table 4, and stabilizers with deviations (Bias) measured within 20% throughout the entire storage period were evaluated as being effective for ensuring the storage stability of the immunoreagent (R2). Table 4 shows the experimental conditions according to the type and concentration of stabilizer.

**Table 4: Experimental Conditions according to the type and concentration of stabilizer**

| **Conditions** | **Control** | **1** | **2** | **3** | **4** | **5** |
|---|---|---|---|---|---|---|
| **stabilizer** | - | 100mM | 100mM | 5% (w/v) | 5% (w/v) | 17mg/mL |
| | | **Trehalose** | **Sucrose** | **DEAE-Dextran** | **Polyacrylic Acid (PAA)** | **Neo Protein Saver (NPS)** |

### 2. Experimental Results

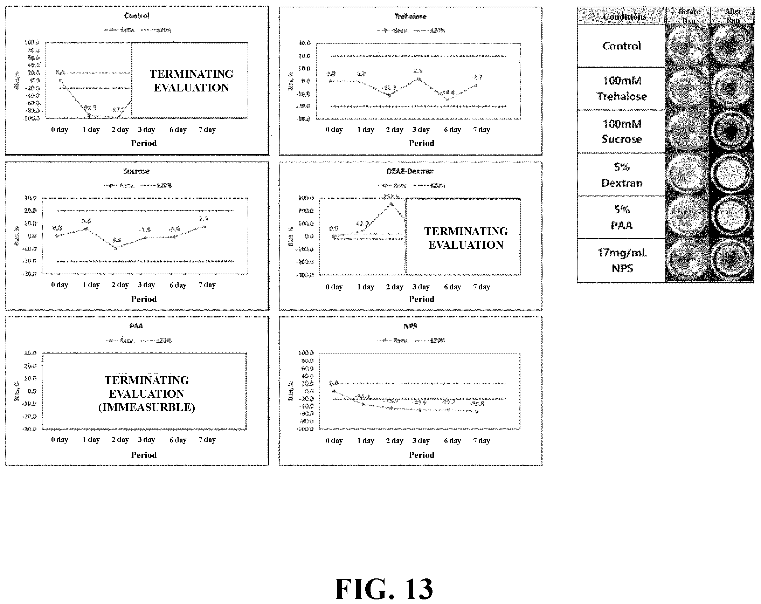

FIG 13 is a graph and diagram showing the results of accelerated stability evaluation for stabilizers added to the Anti-CRP antibody according to an embodiment of the present application.

In the case of the control group (i.e., without the addition of a stabilizer), it was observed that the measurement value decreased by more than 90% compared to the baseline measurement value on day 0, starting from storage day 1. This confirmed that a stabilizer is essential to ensure the storage stability of the immunoreagent (R2).

For the DEAE-Dextran stabilizer, it was visually confirmed that the dried Anti-CRP antibodies did not dissolve properly. Additionally, starting from storage day 1, the measurement value increased by more than 42% compared to the baseline measurement value on day 0.

For the PAA stabilizer, it was visually confirmed that the dried Anti-CRP antibodies did not dissolve properly. And, the immune reaction was not properly conducted, making it impossible to measure the absorbance.

For the NPS stabilizer, it was observed that the deviation (Bias) of the measurement value exceeded the 20% range compared to the baseline measurement value of day 0, starting from storage day 1.

For the Trehalose and Sucrose stabilizers, it was visually confirmed that the dried Anti-CRP antibodies dissolved properly. Additionally, the measurement values remained within the 20% deviation (Bias) range compared to the baseline measurement value on day 0, even up to storage day 7.

According to this experimental example, the stabilizer in one embodiment of the present application may be selected from the group consisting of Trehalose and Sucrose.

### <Experimental Example 5: Evaluation of Accelerated Stability by Stabilizers (2)>

### 1. Experimental Method

To select a stabilizer for ensuring the storage stability of the immunoreagent (R2) containing Anti-CRP antibodies, two candidate stabilizers (Trehalose and Sucrose) with confirmed through Experimental Example 4 storage stability effects were prepared. Each candidate stabilizer was added to latex particles coated with Anti-CRP antibodies and dried. The reagent solution and 3-Level CRP Serum Samples (CRP 10 mg/L, 50 mg/L, 150 mg/L) were added to the dried compositions, and the immune reaction between CRP and Anti-CRP antibodies was carried out. After the immune reaction, the absorbance was measured to obtain the baseline measurement value of storage on day 0.

Each candidate stabilizer was added to latex particles coated with Anti-CRP antibodies, and the dried compositions were stored in an oven at 50°C. The reagent solution was stored separately under refrigeration. At 1-day intervals over a period of 7 days, the reagent solution and 3-Level CRP Serum Samples (CRP 10 mg/L, 50 mg/L, 150 mg/L) were added to the dried compositions containing each candidate stabilizer. The immune reaction between CRP and Anti-CRP antibodies was conducted, and the absorbance after the immune reaction was measured to obtain the measurement values for storage days 1, 2, 3, 4, 5, 6, and 7.

Based on the baseline measurement value and the measurement values obtained during the storage period, deviations (Bias) were calculated.

Additionally, for each condition, the experiment was repeated 5 times, and precision (CV) was calculated.

The two candidate stabilizers are listed below Table 5, and stabilizers with deviations (Bias) measured within 20% throughout the storage period were evaluated as effective for ensuring the storage stability of the immunoreagent (R2). Stabilizers with precision (CV) measured within 10% were evaluated as having excellent precision. Table 5 shows the experimental conditions according to the type and concentration of selected stabilizer.

**Table 5: Experimental Conditions according to the type and concentration of selected stabilizer**

| **Conditions** | **1** | **2** |
|---|---|---|
| **Stabilizers** | 100mM | 100mM |
| | **Trehalose** | **Sucrose** |

### 2. Experimental Results

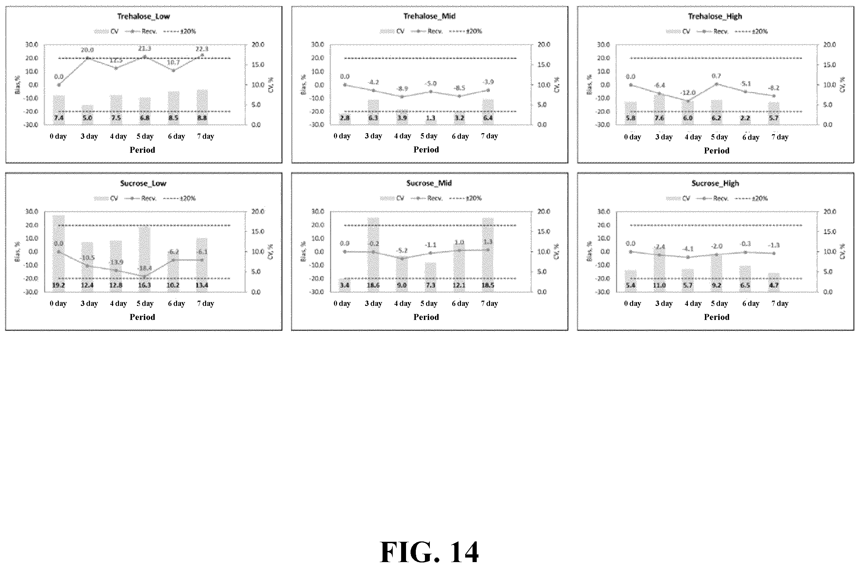

FIG 14 is a graph showing the results of accelerated stability evaluation for stabilizers added to the Anti-CRP antibody according to an embodiment of the present application.

For the stabilizer Trehalose and the CRP Serum (10 mg/L, Low) sample, it was observed that the measurement values on storage days 5 and 7 exceeded the 20% deviation (Bias) range compared to the baseline measurement value on day 0. Furthermore, for Trehalose and the CRP Serum (10 mg/L, Low) sample, the precision (CV) was observed to remain below 10% throughout the entire storage period.

For the stabilizer Trehalose and the CRP Serum (50 mg/L, Mid) sample, it was confirmed that the measurement values remained within the 20% deviation (Bias) range compared to the baseline measurement value on day 0, up to storage day 7. Furthermore, for Trehalose and the CRP Serum (50 mg/L, Mid) sample, the precision (CV) was observed to remain below 10% throughout the entire storage period.

For the stabilizer Trehalose and the CRP Serum (150 mg/L, High) sample, it was confirmed that the measurement values remained within the 20% deviation (Bias) range compared to the baseline measurement value on day 0, up to storage day 7. Furthermore, for Trehalose and the CRP Serum (150 mg/L, High) sample, the precision (CV) was observed to remain below 10% throughout the entire storage period.

For the stabilizer Sucrose and the CRP Serum (10 mg/L, Low) sample, it was confirmed that the measurement values remained within the 20% deviation (Bias) range compared to the baseline measurement value on day 0, up to storage day 7. However, for the stabilizer Sucrose and the CRP Serum (10 mg/L, Low) sample, the precision (CV) was observed to exceed 10% throughout the entire storage period.

For the stabilizer Sucrose and the CRP Serum (50 mg/L, Mid) sample, it was confirmed that the measurement values remained within the 20% deviation (Bias) range compared to the baseline measurement value on day 0, up to storage day 7. However, for the stabilizer Sucrose and the CRP Serum (50 mg/L, Mid) sample, the precision (CV) was observed to exceed 10% on storage days 3, 6, and 7.

For the stabilizer Sucrose and the CRP Serum (150 mg/L, High) sample, it was confirmed that the measurement values remained within the 20% deviation (Bias) range compared to the baseline measurement value on day 0, up to storage day 7. However, for the stabilizer Sucrose and the CRP Serum (150 mg/L, High) sample, the precision (CV) was observed to exceed 10% on storage day 3.

Through this experimental example, it was confirmed that both the Sucrose and Trehalose stabilizers can enhance storage stability with high precision across most CRP concentration ranges. However, additional experiments were conducted to further ensure storage stability and improve precision for certain CRP concentration ranges.

### <Experimental Example 6: Evaluation of Accelerated Stability by Stabilizers (3)>

### 1. Experimental Method

The evaluation was conducted using the same experimental method as in Experimental Example 5, employing a stabilizer that was a mixture of Sucrose and Trehalose. Specifically, a stabilizer composed of a mixture of Sucrose and Trehalose (hereafter referred to as the "mixed stabilizer") was prepared. Furthermore, the mixed stabilizer was added to latex particles coated with Anti-CRP antibodies and dried. The reagent solution and 3-Level CRP Serum Samples (CRP 10 mg/L, 50 mg/L, 150 mg/L) were added to the dried compositions, and the immune reaction between CRP and Anti-CRP antibodies was carried out. After the immune reaction, the absorbance was measured to obtain the baseline measurement value for storage on day 0.

The dried compositions containing the mixed stabilizer and Anti-CRP antibody-coated latex particles were stored in an oven at 50°C. The reagent solution was stored separately under refrigeration. At 1-day intervals over a period of 7 days, the reagent solution and 3-Level CRP Serum Samples (CRP 10 mg/L, 50 mg/L, 150 mg/L) were added to the dried compositions containing the mixed stabilizer. The immune reaction between CRP and Anti-CRP antibodies was conducted, and the absorbance after the immune reaction was measured to obtain the measurement values for storage days 1, 2, 3, 4, 5, 6, and 7.

The deviation (Bias) was calculated based on the baseline measurement value and the measurement values obtained during the storage period.

Additionally, for each CRP concentration condition, the experiment was repeated 5 times, and precision (CV) was calculated.

The mixed stabilizer is as described below Table 6. Table 6 shows the stabilizer used in Experimental Example 6.

Stabilizers with deviations (Bias) measured within 20% throughout the storage period were evaluated as effective for ensuring the storage stability of the immunoreagent (R2). Stabilizers with precision (CV) measured within 10% were evaluated as having excellent precision.

**Table 6: Stabilizer used in Experimental Example 6**

| **Conditions** | 1 |
|---|---|
| **Stabilizers** | 100mM **Trehalose** + 100mM **Sucrose** |

### 2. Experimental Results

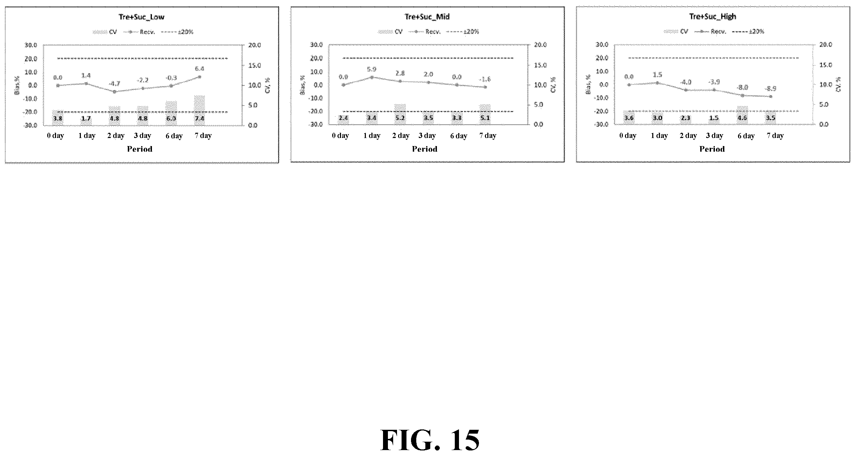

FIG 15 is a graph showing the results of accelerated stability evaluation for stabilizers added to the Anti-CRP antibody according to an embodiment of the present application.

For the mixed stabilizer and the CRP Serum (10 mg/L, Low) sample, it was confirmed that the measurement values remained within the 20% deviation (Bias) range compared to the baseline measurement value on day 0, up to storage day 7. Furthermore, for the mixed stabilizer and the CRP Serum (10 mg/L, Low) sample, the precision (CV) was observed to remain below 10% throughout the entire storage period.

For the mixed stabilizer and the CRP Serum (50 mg/L, Mid) sample, it was confirmed that the measurement values remained within the 20% deviation (Bias) range compared to the baseline measurement value on day 0, up to storage day 7. Furthermore, for the mixed stabilizer and the CRP Serum (50 mg/L, Mid) sample, the precision (CV) was observed to remain below 10% throughout the entire storage period.

For the mixed stabilizer and the CRP Serum (150 mg/L, High) sample, it was confirmed that the measurement values remained within the 20% deviation (Bias) range compared to the baseline measurement value on day 0, up to storage day 7. Furthermore, for the mixed stabilizer and the CRP Serum (150 mg/L, High) sample, the precision (CV) was observed to remain below 10% throughout the entire storage period.

Through this, it was confirmed that using a stabilizer composed of a mixture of Sucrose and Trehalose can enhance storage stability with high precision across all CRP concentration ranges. According to Experimental Example 6, the stabilizer in the preferred embodiment of the present application may include both Trehalose and Sucrose.

Effects of the present invention are not limited to the above-described effects and other effects that are not described may be clearly understood by those skilled in the art from the above detailed descriptions.

### <Experimental Example 7: Evaluation of Accelerated Stability by Stabilizers (4)>

### 1. Experimental Method

A stabilizer composed of a mixture of Sucrose and Trehalose was evaluated for storage stability under modified storage temperature conditions compared to Experimental Example 6. Specifically, a stabilizer composed of a mixture of Sucrose and Trehalose was prepared. Furthermore, the mixed stabilizer was added to latex particles coated with Anti-CRP antibodies and dried. The reagent solution and CRP Control Solutions (Low, High) were each added to the dried compositions to facilitate the immune reaction between CRP and Anti-CRP antibodies. Subsequently, the absorbance after the immune reaction was measured to obtain the baseline measurement value for storage day 0.

Furthermore, the dried compositions were prepared by adding the mixed stabilizer to latex particles coated with Anti-CRP antibodies and stored in an oven at 37°C. In Experimental Example 7, the reagent solution was also stored in the oven at 37°C. At regular intervals over 63 days, the dried compositions containing the mixed stabilizer were added with the reagent solution and CRP Control Solutions (Low, High) to facilitate the immune reaction between CRP and Anti-CRP antibodies. After the immune reaction, the absorbance was measured to obtain measurement values for days 4, 7, 14, 21, 28, 35, 42, 56, and 63.

The deviation (Bias) was calculated based on the baseline measurement value and the measurement values obtained during the storage period. Additionally, for each CRP concentration condition, the experiment was repeated five times, and the precision (CV) was calculated.

The mixed stabilizer is as described below Table 7. Table 7 shows the stabilizer used in Experimental Example 7. Stabilizers with a deviation (Bias) measured within 20% throughout the storage period were evaluated as effective in ensuring the storage stability of the immunoreagent (R2). Stabilizers with a precision (CV) measured within 10% were evaluated as having excellent precision.

**Table 7: Stabilizer used in Experimental Example 7**

| **Conditions** | 1 |
|---|---|
| **Stabilizers** | 100mM **Trehalose** + 100mM **Sucrose** |

### 2. Experimental Results

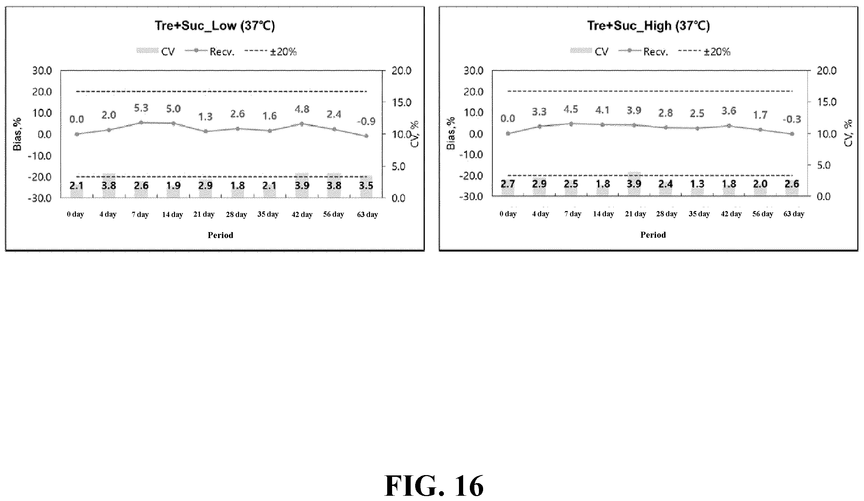

FIG 16 is a graph showing the results of accelerated stability evaluation for stabilizers added to the Anti-CRP antibody according to an embodiment of the present application.

The measurement values for the mixed stabilizer with the CRP Control Solution (Low) sample were confirmed to remain within a 20% deviation (Bias) from the baseline measurement value on day 0, up to storage day 63. Furthermore, for the mixed stabilizer with the CRP Control Solution (Low) sample, the precision (CV) was observed to be below 10% throughout the entire storage period.

The measurement values for the mixed stabilizer with the CRP Control Solution (High) sample were confirmed to remain within a 20% deviation (Bias) from the baseline measurement value on day 0, up to storage day 63. Furthermore, for the mixed stabilizer with the CRP Control Solution (High) sample, the precision (CV) was observed to be below 10% throughout the entire storage period.

This demonstrates that when using a stabilizer composed of a mixture of Sucrose and Trehalose, storage stability can be maintained with high precision even after being stored for over 63 days at a storage temperature of 37°C. According to Experimental Example 7, the stabilizer in the preferred embodiment of the present application may include both Trehalose and Sucrose. More preferably, the stabilizer may include Trehalose and Sucrose in substantially equal concentrations.

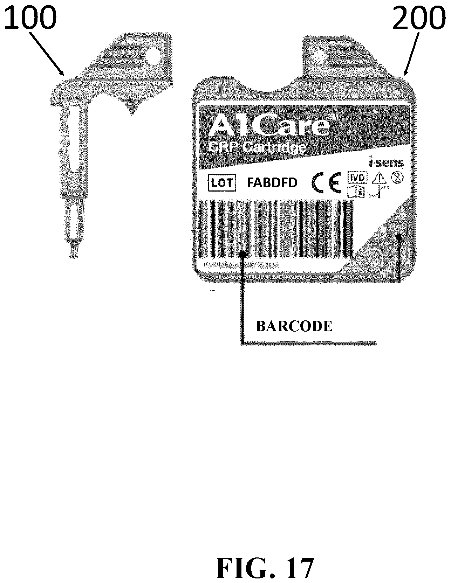

FIG 17 is a diagram showing identification information included in the CRP quantitative analysis kit according to an embodiment of the present application.

The CRP quantitative analysis kit 10 according to one embodiment of the present application may include identification information for recognizing a biological sample. For example, the CRP quantitative analysis kit 10 may include identification information (e.g., information in the form of a barcode) on one surface of the main cartridge 200 to identify the type of biological sample.

The quantitative analysis apparatus(or quantitative analysis device), described later, may acquire identification information to recognize the type of biological sample to be analyzed by the CRP quantitative analysis kit 10. For instance, the quantitative analysis device may determine, based on a barcode, that the biological sample for analysis is CRP. In this case, the quantitative analysis device may be configured to perform quantitative analysis of the biological sample by executing a pre-stored analysis protocol associated with the recognized type, based on the recognized type of the biological sample. For example, the quantitative analysis device may be implemented to execute a pre-stored CRP analysis protocol based on recognizing that the type of biological sample to be analyzed is CRP, thereby performing quantitative analysis of CRP.

Meanwhile, FIG 17 illustrates identification information in the form of a barcode as an example. However, this is merely an example, and any suitable form of identification information may be provided at any suitable location on the CRP quantitative analysis kit 10.

The quantitative analysis device according to an embodiment of the present disclosure may be configured to perform quantitative analysis of biological samples (e.g., 1,5-AG, glycated albumin, CRP, etc.). Furthermore, the quantitative analysis device may include a communication module (which may also be referred to as a transceiver), a memory, and/or a processor.

The communication module of the quantitative analysis device may communicate with any external device or external server. For example, the quantitative analysis device may transmit quantitative analysis results to an external device or external server via the communication module.

The quantitative analysis device may access a network through the communication module to transmit or receive various types of data. The communication module may largely include a wired-type communication module and a wireless-type communication module. Since the wired-type communication module and the wireless-type communication module have their own advantages and disadvantages, in some cases, the wired-type communication module and the wireless-type communication module may be provided together as the quantitative analysis device. Here, in the case of the wireless-type communication module, a wireless local area network (WLAN) type communication method such as Wi-Fi may be mainly used. Alternatively, in the case of the wireless-type communication module, cellular communication, such as long-term evolution (LTE) or 5G communication methods, may be used. However, a wireless communication protocol is not limited to the above-described example, and any appropriate wireless type of communication method may be used. In the case of the wired-type communication module, a local area network (LAN) or Universal Serial Bus (USB) communication is a representative example, but other methods may be used.

Various types of information may be stored in the memory of the quantitative analysis device. Various types of data may be temporarily or semi-permanently stored in the memory. Examples of the memory may include a hard disk drive (HDD), a solid state drive (SSD), a flash memory, a read-only memory (ROM), a random access memory (RAM), etc. The memory may be provided to be embedded in the e quantitative analysis device or in a detachable form. Various types of data necessary for the operation of the quantitative analysis device, including an operating program (OS) for driving the quantitative analysis device or a program for operating each component of the quantitative analysis device, may be stored in the memory.

The processor may control the overall operation of the quantitative analysis device. For example, the quantitative analysis device may control its overall operations, including actions such as recognizing the identification information of a biological sample, executing a corresponding analysis protocol based on the recognized identification information, and/or performing quantitative analysis of the biological sample according to the analysis protocol. Specifically, the processor may load and execute a program for the overall operation of the quantitative analysis device from the memory. The processor may be implemented as an application processor (AP), a central processing unit (CPU), a microcontroller unit (MCU), or a similar device depending on hardware, software, or a combination thereof. In this case, the processor may be provided in the form of an electronic circuit that processes electrical signals and performs a control function in hardware, and may be provided in the form of a program or code that drives a hardware circuit in software.

According to an embodiment of the present application, the CRP quantitative analysis kit, CRP quantitative analysis method, and/or the apparatus for performing the same may provide a hemolyzing reagent capable of performing quantitative analysis of CRP across a wide concentration range with high precision, without affecting the immune reaction.

According to an embodiment of the present application, the CRP quantitative analysis kit, CRP quantitative analysis method, and/or the apparatus for performing the same may provide a stabilizer that allows the solid-phase dried Anti-CRP antibody to sufficiently redissolve for participation in immune reactions, enables highly precise and accurate quantitative analysis of CRP across a wide concentration range, and enhances storage stability.

Effects of the present invention are not limited to the above-described effects and other effects that are not described may be clearly understood by those skilled in the art from the above detailed descriptions.

Features, structures, and effects described in the above-described exemplary embodiments are included in at least one exemplary embodiment of the present invention, but are not necessarily limited to only one exemplary embodiment. Furthermore, features, structures, and effects described in each embodiment can be combined or modified and implemented in other embodiments by one of ordinary skill in the art to which the embodiments belong. Therefore, it should be interpreted that contents related to such combinations and modifications are included in the scope of the present invention.

Further, while the present invention has been particularly described with reference to embodiments, the embodiments are only exemplary embodiments of the present invention and the present invention is not intended to be limited thereto. It will be understood by those skilled in the art that modifications and applications in other forms may be made without departing from the spirit and scope of the present invention. That is, each element specifically shown in the embodiments may be modified and embodied. In addition, it should be understood that differences related to these modifications and applications are within the scope of the present invention as defined in the appended claims.

## Claims

1. A kit for quantitative analysis of C-reactive protein (CRP), comprising:
a first composition including a hemolytic reagent for hemolyzing at least a portion of blood cells in a blood sample; and
a second composition including an anti-CRP antibody for antigen-antibody reaction of CRP contained in the hemolyzed sample,
wherein the CRP quantitative analysis kit for quantitative analysis of CRP comprises at least one reagent fixing part,
the first composition and the second composition are independently fixed to the reagent fixing part, respectively, and
the second composition further comprises a stabilizer for the stability of the anti-CRP antibody.

2. The kit for quantitative analysis of CRP according to claim 1, wherein the hemolytic reagent is selected from the group consisting of Sodium Deoxycholate (SDO) and Saponin.

3. The kit for quantitative analysis of CRP according to claim 1, wherein the hemolytic reagent is Sodium Deoxycholate (SDO).

4. The kit for quantitative analysis of CRP according to claim 1, wherein the stabilizer is selected from the group consisting of Trehalose and Sucrose.

5. The kit for quantitative analysis of CRP according to claim 1, wherein the stabilizer includes both Trehalose and Sucrose.

6. The kit for quantitative analysis of CRP according to claim 5, wherein the stabilizer comprises Trehalose and Sucrose in substantially the same concentration ratio.

7. The kit for quantitative analysis of CRP according to Claim 1, wherein the at least one reagent fixing part comprises:
a first reagent fixing part located in a first compartmental region of the CRP quantitative analysis kit, and
a second reagent fixing part located in a second compartmental region partitioned from the first compartmental region,
wherein the first composition is fixed to the first reagent fixing part, and the second composition is fixed to the second reagent fixing part.

8. The kit for quantitative analysis of CRP according to claim 7,
wherein the CRP quantitative analysis kit includes a solution cell disposed in the first compartmental region,
the solution cell stores a reaction buffer, and
when a sample collector containing the blood sample is inserted into a receiving part of the CRP quantitative analysis kit, the reaction buffer stored in the solution cell and the blood sample contained in the sample collector flow into a mixing region where the first reagent fixing part in the first compartmental region is disposed,
so that at least a portion of blood cells in the blood sample are hemolyzed in the mixing region.

9. The kit for quantitative analysis of CRP according to claim 8, wherein the reaction buffer comprises Glycine, Sodium Chloride, Sodium Azide, Ethylenediaminetetraacetic acid disodium salt dihydrate, and Bovine Serum Albumin (BSA).

10. The kit for quantitative analysis of CRP according to claim 8,
wherein the CRP quantitative analysis kit further comprises a flow path connecting the first compartmental region and the second compartmental region, and
the sample, in which at least a portion of the blood cells are hemolyzed, flows from the mixing region in the first compartmental region to the second compartmental region through the flow path as the CRP quantitative analysis kit rotates by a predetermined angle due to an applied external force.

11. The kit for quantitative analysis of CRP according to claim 10,
wherein the CRP quantitative analysis kit further comprises a measurement unit for quantifying CRP contained in the blood sample, and
the sample, in which at least a portion of the blood cells are hemolyzed, passes through the measurement unit via the flow path while moving from the mixing region in the first compartmental region to the second reagent fixing part in the second compartmental region.

12. The kit for quantitative analysis of CRP according to claim 11,
wherein the quantification of CRP is performed based on a first turbidity measured through the measurement unit before the antigen-antibody reaction and a second turbidity measured through the measurement unit after the antigen-antibody reaction performed at the second reagent fixing part.

13. The kit for quantitative analysis of CRP according to claim 1, wherein the second composition comprises the stabilizer for immobilizing the anti-CRP antibody in a solid state on the reagent fixing part.

14. The kit for quantitative analysis of CRP according to claim 1, wherein the anti-CRP antibody is provided in a form coated on latex particles.
